# EUROPEAN PATENT SPECIFICATION

(11) **EP 4 286 828 B1**
(45) Date of publication and mention of the grant of the patent: **17.12.2025**
(21) Application number: 23175464.9
(22) Date of filing: 25.05.2023
(51) Int. Cl.: G01N 15/12, G01N 15/10, G01N 15/0227, G01N 15/02, G01N 15/14, G01N 33/493

(54) **PRESERVATION LIQUID FOR CALCIUM OXALATE DIHYDRATE CRYSTALS AND PRESERVATION METHOD**
KONSERVIERUNGSFLÜSSIGKEIT FÜR CALCIUMOXALATDIHYDRATKRISTALLE UND KONSERVIERUNGSVERFAHREN
LIQUIDE DE CONSERVATION POUR CRISTAUX DE DIHYDRATE D'OXALATE DE CALCIUM ET PROCÉDÉ DE CONSERVATION

(30) Priority: 31.05.2022 JP 2022088515; 16.05.2023 JP 2023080983
(43) Date of publication of application: 06.12.2023
(73) Proprietor: ARKRAY, Inc., Kyoto-shi, Kyoto 601-8045 (JP)
(72) Inventor: HIROTA, Takuma, Kyoto-shi, 602-0008 (JP); NAKAO, Atsushi, Kyoto-shi, 602-0008 (JP)
(74) Representative: Pitchford, James Edward

(56) References cited:
- JP-A- S 641 927
- US-A- 4 331 862
- DESMARS J F ET AL: "Dissolution and growth of calcium oxalate monohydrate I. Effect of magnesium and pH", BIOCHIMICA ET BIOPHYSICA ACTA, ELSEVIER, AMSTERDAM, NL, vol. 313, no. 2, 28 July 1973 (1973-07-28), pages 256 - 267, XP025793535, ISSN: 0304-4165, [retrieved on 19730728], DOI: 10.1016/0304-4165(73)90025-1

## Description

### TECHNICAL FIELD

The present disclosure relates to a preservation liquid for calcium oxalate dihydrate crystals, and a method for preserving calcium oxalate dihydrate crystals. The present disclosure also relates to a quality control liquid for use in the examination of urine sediments, which liquid is used particularly for the quality control on crystal items in a urine sediment analysis using a urine sediment analyzer based on microscopic observation.

### BACKGROUND ART

A urine sediment analyzer based on a microscope is used for evaluating the presence of various analyte species in a urine sample, based on the forms of sediments in the urine sample. Quality control is required in order for the urine sediment analyzer to accurately detect and classify the analyte species based on their forms.

Non-patent Document 1 discloses a control urine for urine sediment examination which contains erythrocytes and leukocytes separated from human-derived blood and immobilized with glutaraldehyde, as a quality control liquid intended for use in quality control.

Further, Patent Document 1 discloses a quality control liquid which contains: a cancer cell as a substance that indicates the form of a non-squamous epithelial cell in urine; an algae cell as a substance that indicates the form of an abnormal cast in urine; a yeast cell as a substance that indicates the form of a yeast cell in urine; and egg white as a substance that indicates the form of mucus in urine.

### PRIOR ART DOCUMENTS

### PATENT DOCUMENTS

[Patent Document 1] JP2020-531852A
[Patent Document 2] JP2019-066461A

### NON-PATENT DOCUMENTS

[Non-patent Document 1] Nobuko Imai et al., "Preparation and utility of control urine for urinary sediment examination", Hygiene Examination, Vol. 39, Issue 1, 1990, p. 55 to 61

### SUMMARY

In the urine sediment analysis using a urine sediment analyzer based on a microscope, disclosed in Patent Document 1 or Non-patent Document 1, it is possible to perform quality control in the analysis of erythrocytes, leukocytes, non-squamous epithelial cells, abnormal casts, yeast cells and mucus that can be present in urine, using the quality control liquid. However, quality control cannot be performed on the items of substances that can be present in urine, other than those mentioned above, which has been a problem. In particular, there has been no report on a quality control liquid for performing quality control on the items of calcium oxalate dihydrate crystals (hereinafter, also simply referred to as "crystals") that can be present in urine.

Calcium oxalate dihydrate is present as octahedron-shaped crystals in urine. However, it has been discovered that calcium oxalate dihydrate crystals decompose when preserved in a buffer solution for a certain period of time, regardless of whether it is artificially or naturally derived.

In a first aspect the present invention relates to the use of a solution comprising an anionic surfactant or a non-ionic surfactant for preserving calcium oxalate dihydrate crystals. In another aspect the present invention relates to a method for preserving calcium oxalate dihydrate crystals. A solution comprising stabilized calcium oxalate dihydrate crystals can be used on crystal items in a urine sediment analysis using a urine sediment analyzer.

The present inventors have found out that it is possible to increase the preservation stability of the crystal shape of calcium oxalate dihydrate in a solution, by allowing an anionic surfactant or a non-ionic surfactant to coexist with calcium oxalate dihydrate crystals. Further, the present inventors have found out that it is possible to provide a quality control liquid for calcium oxalate dihydrate crystals, which can be used in a urine sediment analysis using a urine sediment analyzer.

Specifically, the present invention is defined in the appended claims. Details of certain embodiments are set out in the dependent claims.

### EFFECTS

The present invention provides the use of a preservation liquid for calcium oxalate dihydrate crystals and a method for preserving calcium oxalate dihydrate crystals, which allow for stably preserving calcium oxalate dihydrate crystals even in a solution. Solutions comprising preserved calcium oxalate dihydrate crystals can be used for performing an analysis on the items of crystals that can be present in urine, with a urine sediment analyzer.

### BRIEF DESCRIPTION OF THE DRAWINGS

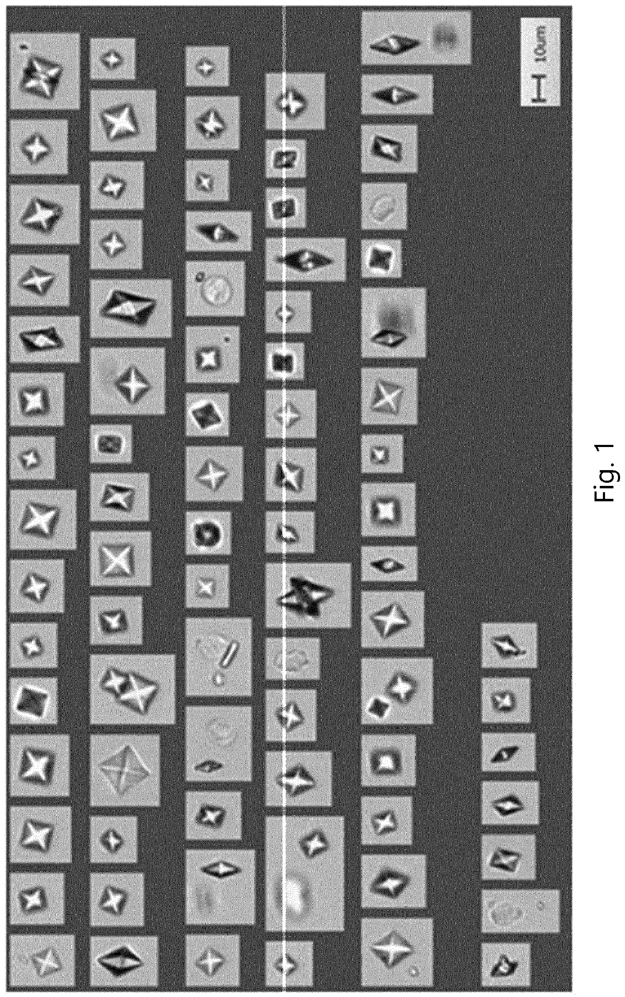

FIG. 1 shows images (photographs) illustrating the forms of calcium oxalate dihydrate crystals described in Example 1, obtained by a urine sediment analyzer based on a microscope.

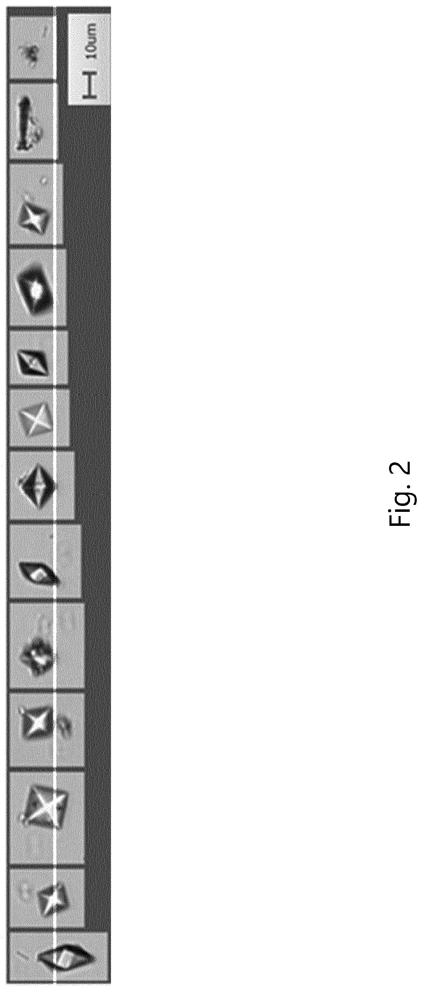

FIG. 2 shows images (photographs) illustrating the forms of calcium oxalate dihydrate crystals described in Reference Example 1, obtained by the urine sediment analyzer based on a microscope.

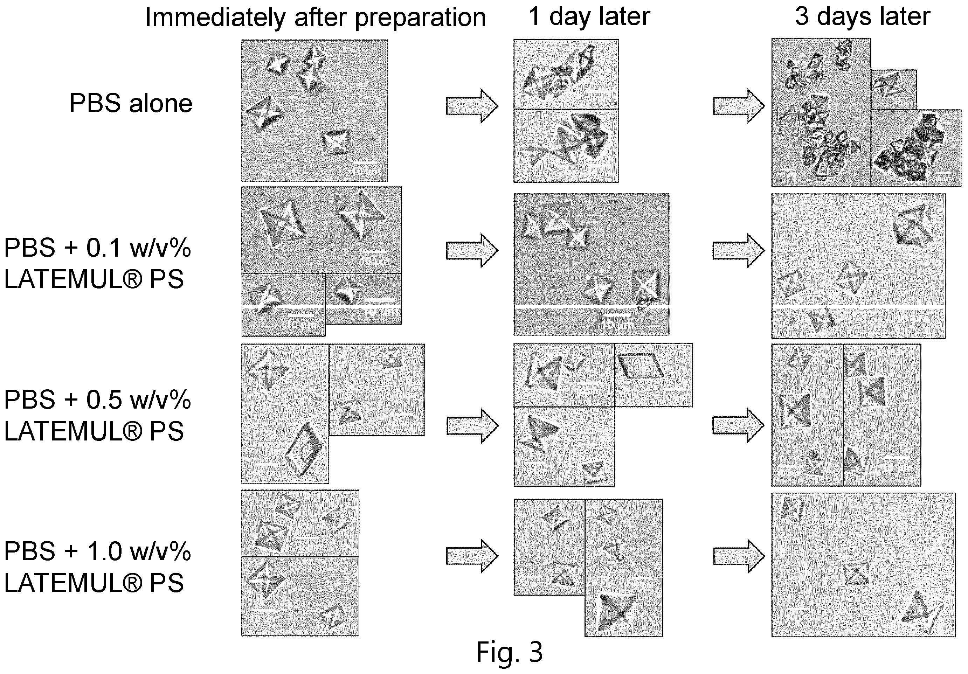

FIG. 3 shows images (photographs) illustrating the forms of calcium oxalate dihydrate crystals described in Example 2-1 and Example 2-2, obtained by microscopic observation, at time points immediately after the preparation of each quality control liquid containing LATEMUL^{®} PS, and one day and three days after allowing each quality control liquid to stand and to be preserved at room temperature.

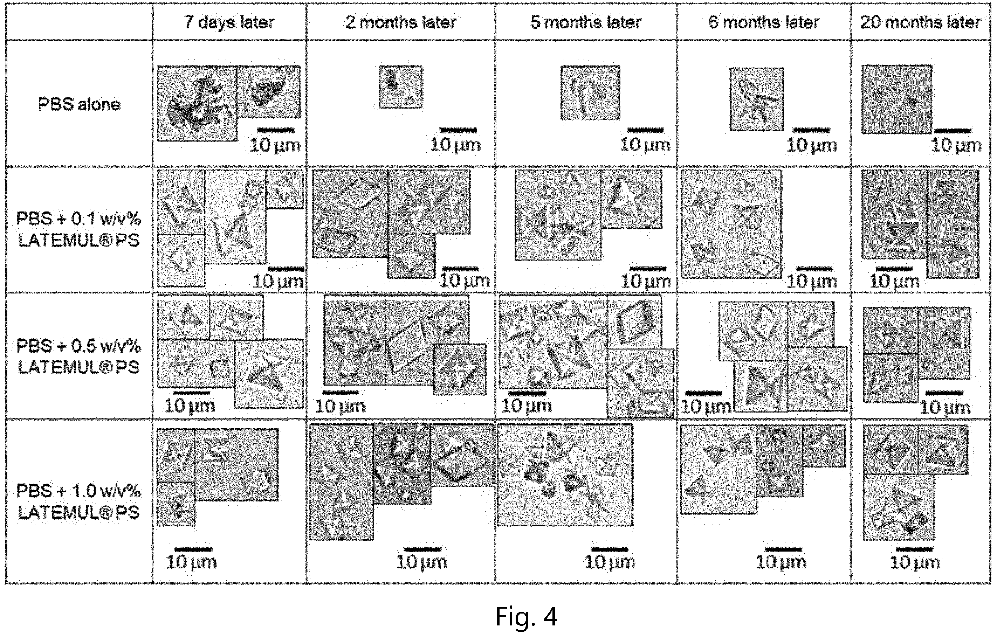

FIG. 4 shows images (photographs) illustrating the forms of calcium oxalate dihydrate crystals described in Example 2-1, obtained by microscopic observation, at time points seven days, two months, five months, six months and 20 months after allowing each quality control liquid containing LATEMUL^{®} PS to stand and to be preserved at room temperature.

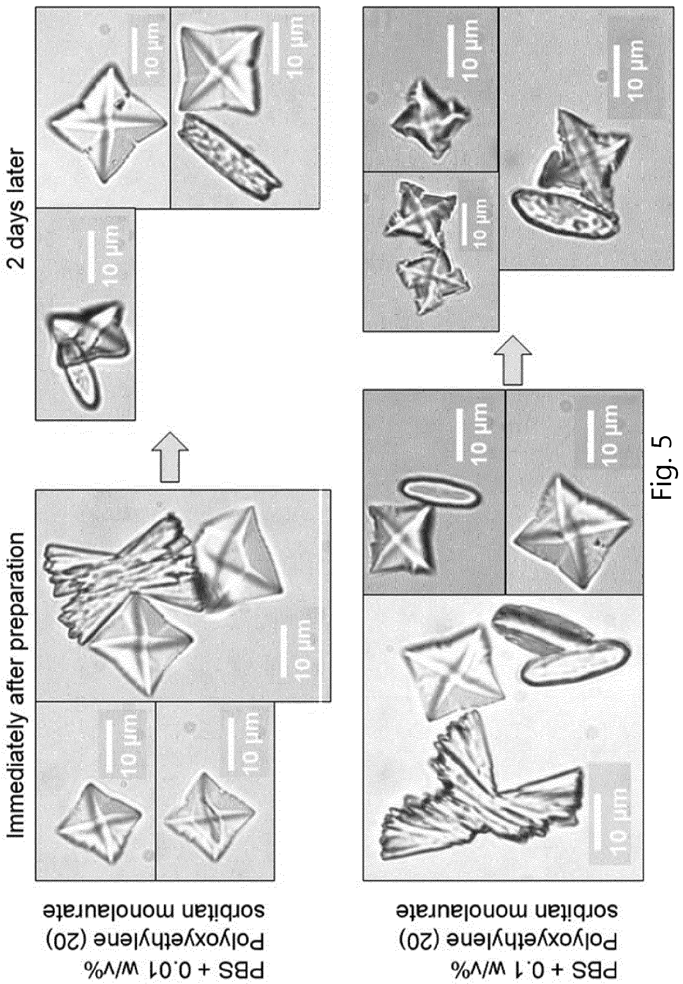

FIG. 5 shows images (photographs) illustrating the forms of calcium oxalate dihydrate crystals described in Example 3, obtained by microscopic observation, at time points immediately after the preparation of each quality control liquid containing polyoxyethylene (20) sorbitan monolaurate, and two days after allowing each quality control liquid to stand and to be preserved at room temperature.

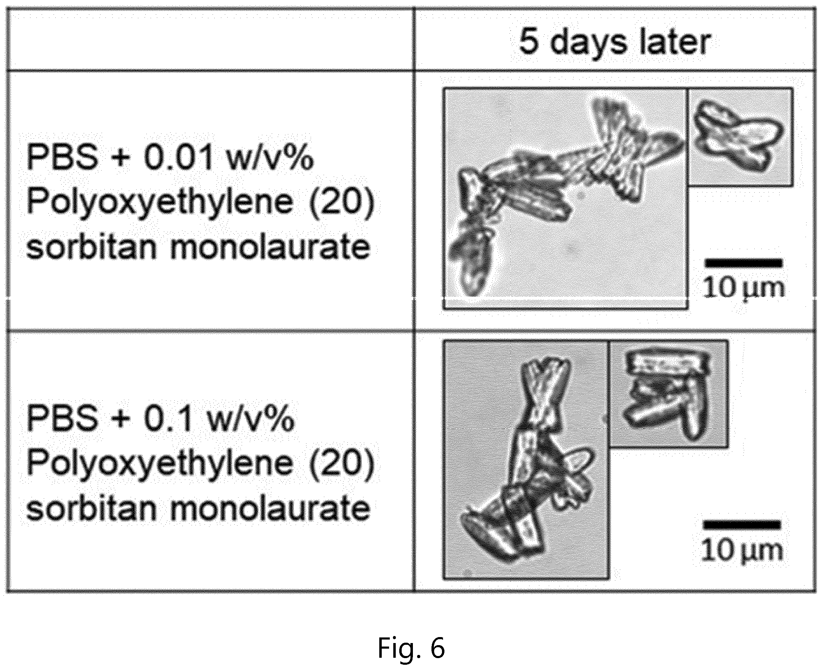

FIG. 6 shows images (photographs) illustrating the forms of calcium oxalate dihydrate crystals described in Example 3, obtained by microscopic observation, at time points five days after allowing each quality control liquid containing polyoxyethylene (20) sorbitan monolaurate to stand and to be preserved at room temperature.

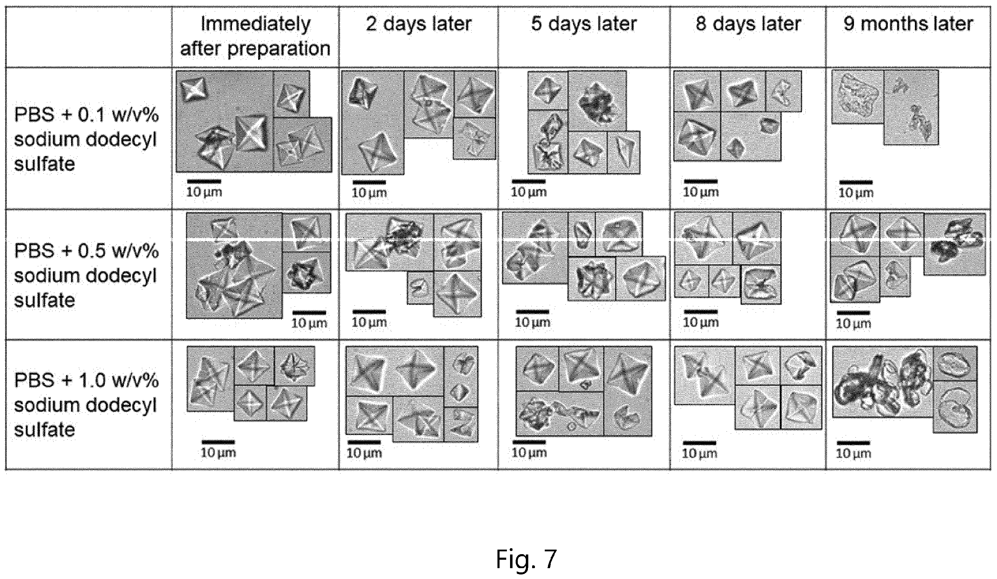

FIG. 7 shows images (photographs) illustrating the forms of calcium oxalate dihydrate crystals described in Example 4, obtained by microscopic observation, at time points immediately after the preparation of each quality control liquid containing sodium dodecyl sulfate (SDS), and two days, five days, eight days and nine months after allowing each quality control liquid to stand and to be preserved at room temperature.

### DETAILED DESCRIPTION OF EMBODIMENTS

### < Preservation Liquid for Calcium Oxalate Dihydrate Crystals >

One embodiment of the present disclosure is a preservation liquid for calcium oxalate dihydrate crystals, which is a solution containing an anionic surfactant or a non-ionic surfactant.

The above-described solution can have a pH of from 7.0 to 7.8, and preferably has a pH of from 7.2 to 7.6. The pH of the preservation liquid is usually within the range described above.

The solution preferably contains a buffer. The solution may contain a single kind, or two or more kinds of buffers. A commercially available buffer can also be used. The buffer may be, for example, an inorganic buffer or an organic buffer. Of these, an inorganic buffer is preferred.

The inorganic buffer is a buffer derived from an inorganic acid. The inorganic buffer may be, for example, a phosphate buffer or a carbonate buffer. Of these, a phosphate buffer is preferred. Examples of the phosphate buffer include phosphoric acid, and salts thereof, such as sodium dihydrogen phosphate, disodium hydrogen phosphate, trisodium phosphate, potassium dihydrogen phosphate, dipotassium hydrogen phosphate and tripotassium phosphate. Of these, a phosphoric salt is preferred. Examples of the carbonate buffer include carbonic acid, and salts thereof, such as sodium hydrogen carbonate, sodium carbonate, ammonium carbonate, potassium carbonate, calcium carbonate, potassium hydrogen carbonate and magnesium carbonate.

The organic buffer is a buffer derived from an organic acid or an organic base. The organic buffer may be, for example, a citrate buffer, an acetate buffer or a Tris buffer.

The solution may be, for example, a phosphate buffered saline (PBS) or a pooled urine. The pooled urine may be the pooled urine disclosed in Non-patent Document 1 as a dispersion solvent.

The anionic surfactant may be preferably, for example, a carboxylate salt surfactant, a sulfate ester salt surfactant, a sulfonate salt surfactant or a phosphate ester salt surfactant. Of these, a sulfonate salt surfactant is preferred. Examples of the sulfonate salt surfactant include alkane sulfonate salts, alkylbenzene sulfonate salts and olefin sulfonate salts. Of these, an alkane sulfonate salt is preferred. The alkane in the alkane sulfonate salt preferably has from 10 to 20 carbon atoms, and the alkane sulfonate salt may be, for example, a sodium salt or a potassium salt. More specifically, it is possible to use sodium alkane sulfonate, such as one commercially available under the name of LATEMUL^{®} PS (manufactured by Kao Corporation).

**In** addition, examples of the carboxylate salt surfactant include soap, N-acylamino acid salts, polyoxyethylene or polyoxypropylene alkyl ether carboxylate salts and acylated peptides; examples of the sulfate ester salt surfactant include sulfated oils, alkyl sulfate salts, alkyl ether sulfate salts, polyoxyethylene or polyoxypropylene alkyl allyl ether sulfate salts and alkyl amide sulfate salts; and examples of the phosphate ester salt surfactant include alkyl phosphate salts and polyoxyethylene or polyoxypropylene alkyl allyl ether phosphate salts.

The concentration of the anionic surfactant contained in the preservation liquid can be adjusted to a final concentration of from 0.01 to 1 w/v%, preferably a final concentration of from 0.1 to 1 w/v%, and more preferably a final concentration of from 0.1 to 0.5 w/v%.

Examples of the non-ionic surfactant include sorbitan fatty acid esters, glycerin fatty acid esters, polyglycerin fatty acid esters, propylene glycol fatty acid esters, polyethylene glycol fatty acid esters, sucrose fatty acid esters, polyoxyethylene fatty acid esters, polyoxyethylene sorbitan fatty acid esters, polyoxyethylene alkyl ethers, polyoxyethylene sorbitol fatty acid ethers, polyoxyethylene glycerin fatty acid ethers, polyoxyethylene propylene glycol fatty acid ethers, polyoxyethylene castor oils, polyoxyethylene hydrogenated castor oils, polyoxyethylene hydrogenated castor oil fatty acid esters, polyoxyethylene phytostanol ethers, polyoxyethylene phytosterol ethers, polyoxyethylene cholestanol ethers and polyoxyethylene cholesteryl ethers. Of these, a polyoxyethylene sorbitan fatty acid ester is preferred. Examples of the polyoxyethylene sorbitan fatty acid ester include polyoxyethylene sorbitan monolaurate, polyoxyethylene sorbitan monomyristate, polyoxyethylene sorbitan monopalmitate, polyoxyethylene sorbitan monostearate and polyoxyethylene sorbitan monobehenate. Of these, polyoxyethylene sorbitan monolaurate (such as one commercially available under the name of Tween^{®} 20) is preferred.

The concentration of the non-ionic surfactant contained in the preservation liquid can be adjusted to a final concentration of from 0.01 to 1 w/v%, preferably a final concentration of from 0.1 to 1 w/v%, and more preferably a final concentration of from 0.1 to 0.5 w/v%.

The preservation liquid according to the present embodiment may further include an additional component(s) as long as the effects of the present disclosure are not impaired. The additional component(s) may be, for example, a pH adjusting agent, an osmotic pressure regulator, a component to be measured with a test paper for a urine qualitative test, and/or a main component (component dissolved in urine) constituting the urine matrix.

Examples of the pH adjusting agent include HCl and NaCl. Examples of the osmotic pressure regulator include salts, such as KCl and NaCl. Examples of the component to be measured with a test paper for a urine qualitative test include glucose, proteins (such as albumin and the like), bilirubin, urobilinogen, hemoglobin, ketone bodies, nitrous acid, and enzymes (such as elastase and the like). Examples of the main component constituting the urine matrix include salts (such as KCl and NaCl), proteins (such as albumin and Tamm-Horsfall Protein), uric acid and urea. Further, the preservation liquid is not prohibited from containing a cationic surfactant and an amphoteric surfactant, as long as the effects of the present disclosure are not impaired.

In the preservation liquid according to the present embodiment, it is preferred not to further add a solvent such as water to the solution, from the viewpoint of avoiding effects such as causing the calcium oxalate dihydrate crystals in the preservation liquid to be more susceptible to collapse, and the like. From the same points of view, it is preferred not to dilute the preservation liquid with a solvent such as water. Even in the case of diluting the preservation liquid, it is preferred that the pH of the preservation liquid after dilution achieve a pH of from 7.0 to 7.8, and that the concentrations of the anionic surfactant and the non-ionic surfactant in the preservation liquid after dilution achieve a final concentration of from 0.01 to 1 w/v%.

The preservation liquid according to the present embodiment can be obtained by mixing the solution, and an anionic surfactant or a non-ionic surfactant.

### < Quality Control Liquid >

One embodiment of the present disclosure is a quality control liquid for use in a urine sediment analyzer, wherein the quality control liquid includes calcium oxalate dihydrate crystals, and preferably, further includes the preservation liquid described in the section of < Preservation Liquid for Calcium Oxalate Dihydrate Crystals > described above.

The quality control liquid according to the present embodiment is preferably a quality control liquid for use in a urine sediment analyzer, particularly, a urine sediment analyzer, wherein the quality control liquid is for controlling the measurement accuracy of the particle number concentration of urine sediment crystals.

The definition of the quality control liquid includes a quality control liquid which is used for the quality control of the particle number concentration of crystals, in the analysis on crystal items using a urine sediment analyzer based on a microscope. The quality control liquid contains calcium oxalate dihydrate crystals, and preferably further contains an anionic surfactant or a non-ionic surfactant.

The quality control liquid can have a pH of from 7.0 to 7.8, and preferably has a pH of from 7.2 to 7.6.

In the quality control liquid according to the present disclosure, calcium oxalate dihydrate is usually present as crystals, and the crystals preferably include octahedron-shaped crystals. The calcium oxalate dihydrate crystals can be obtained by a conventional method, specifically, by the method described in Examples to be described later, but not particularly limited thereto.

The particle number concentration of the calcium oxalate dihydrate crystals contained in the quality control liquid is preferably from 0.1 to 300 crystals/µL.

The calcium oxalate dihydrate crystals contained in the quality control liquid preferably have a diameter of from 5 to 20 µm (5 µm or more). The diameter of each calcium oxalate dihydrate crystal as used herein is defined by the diameter of its circumscribed sphere.

The concentration of the anionic surfactant contained in the quality control liquid can be adjusted to a final concentration of from 0.01 to 1 w/v%, preferably a final concentration of from 0.1 to 1 w/v%, and more preferably a final concentration of from 0.1 to 0.5 w/v%.

The concentration of the non-ionic surfactant contained in the quality control liquid can be adjusted to a final concentration of from 0.01 to 1 w/v%, preferably a final concentration of from 0.1 to 1 w/v%, and more preferably a final concentration of from 0.1 to 0.5 w/v%.

The quality control liquid according to the present embodiment may further include an additional component(s) as long as the effects of the present disclosure are not impaired. The additional component(s) may be, for example, a pH adjusting agent, an osmotic pressure regulator, a component to be measured with a test paper for a urine qualitative test, and/or a main component (component dissolved in urine) constituting the urine matrix.

Examples of the pH adjusting agent include HCl and NaCl. Examples of the osmotic pressure regulator include salts, such as KCl and NaCl. Examples of the component to be measured with a test paper for a urine qualitative test include glucose, proteins (such as albumin and the like), bilirubin, urobilinogen, hemoglobin, ketone bodies, nitrous acid, and enzymes (such as elastase and the like). Examples of the main component constituting the urine matrix include salts (such as KCl and NaCl), proteins (such as albumin and Tamm-Horsfall Protein), uric acid and urea. Further, the quality control liquid is not prohibited from containing a cationic surfactant and an amphoteric surfactant, as long as the effects of the present disclosure are not impaired.

It is preferred not to dilute the quality control liquid according to the present embodiment with a solvent such as water, from the viewpoint of avoiding effects such as causing the calcium oxalate dihydrate crystals in the quality control liquid to be more susceptible to collapse, and the like. Even in the case of diluting the quality control liquid, it is preferred that the pH of the quality control liquid after dilution achieve a pH of from 7.0 to 7.8, and that the concentration of the anionic surfactant or the non-ionic surfactant in the quality control liquid after dilution achieve a final concentration of from 0.01 to 1 w/v%. From the same points of view, it is preferred not to further add a solvent such as water to the preservation liquid.

The quality control liquid according to the present embodiment can be obtained by mixing the preservation liquid described in the section of < Preservation Liquid for Calcium Oxalate Dihydrate Crystals > described above, and calcium oxalate dihydrate crystals. The calcium oxalate dihydrate crystals are preferably used after being washed with an aqueous solution containing an anionic surfactant or a non-ionic surfactant.

### < Method for Preserving Calcium Oxalate Dihydrate crystals >

One embodiment of the present disclosure is a method for preserving calcium oxalate dihydrate crystals, the method including preserving calcium oxalate dihydrate crystals in a solution containing an anionic surfactant or a non-ionic surfactant.

The above-described solution can have a pH of from 7.0 to 7.8, and preferably has a pH of from 7.2 to 7.6.

The solution preferably contains a buffer. The solution may contain a single kind, or two or more kinds of buffers. A commercially available buffer can also be used. The buffer may be, for example, an inorganic buffer or an organic buffer. Of these, an inorganic buffer is preferred.

The inorganic buffer is a buffer derived from an inorganic acid. The inorganic buffer may be, for example, a phosphate buffer or a carbonate buffer. Of these, a phosphate buffer is preferred. Examples of the phosphate buffer include phosphoric acid, and salts thereof, such as sodium dihydrogen phosphate, disodium hydrogen phosphate, trisodium phosphate, potassium dihydrogen phosphate, dipotassium hydrogen phosphate and tripotassium phosphate. Of these, a phosphoric salt is preferred. Examples of the carbonate buffer include carbonic acid, and salts thereof, such as sodium hydrogen carbonate, sodium carbonate, ammonium carbonate, potassium carbonate, calcium carbonate, potassium hydrogen carbonate and magnesium carbonate.

The organic buffer is a buffer derived from an organic acid or an organic base. The organic buffer may be, for example, a citrate buffer, an acetate buffer or a Tris buffer.

The solution may be, for example, a phosphate buffered saline (PBS) or a pooled urine. The pooled urine may be the pooled urine disclosed in Non-patent Document 1 as a dispersion solvent.

The particle number concentration of the calcium oxalate dihydrate crystals to be preserved in the solution is preferably from 0.1 to 300 crystals/µL.

The concentration of the anionic surfactant contained in the solution can be adjusted to a final concentration of from 0.01 to 1 w/v%, preferably a final concentration of from 0.1 to 1 w/v%, and more preferably a final concentration of from 0.1 to 0.5 w/v%.

The concentration of the non-ionic surfactant contained in the solution can be adjusted to a final concentration of from 0.01 to 1 w/v%, preferably a final concentration of from 0.1 to 1 w/v%, and more preferably a final concentration of from 0.1 to 0.5 w/v%.

The solution may further include an additional component(s) as long as the effects of the present disclosure are not impaired. The additional component(s) may be, for example, a pH adjusting agent, an osmotic pressure regulator, a component to be measured with a test paper for a urine qualitative test, and/or a main component (component dissolved in urine) constituting the urine matrix.

Examples of the pH adjusting agent include HCl and NaCl. Examples of the osmotic pressure regulator include salts, such as KCl and NaCl. Examples of the component to be measured with a test paper for a urine qualitative test include glucose, proteins (such as albumin and the like), bilirubin, urobilinogen, hemoglobin, ketone bodies, nitrous acid, and enzymes (such as elastase and the like). Examples of the main component constituting the urine matrix include salts (such as KCl and NaCl), proteins (such as albumin and Tamm-Horsfall Protein), uric acid and urea. Further, the solution is not prohibited from containing a cationic surfactant and an amphoteric surfactant, as long as the effects of the present disclosure are not impaired.

In the preservation method according to the present embodiment, it is preferred not to further add a solvent such as water to the solution, from the viewpoint of avoiding effects such as causing the calcium oxalate dihydrate crystals in the solution to be more susceptible to collapse, and the like. From the same points of view, it is preferred not to dilute the solution with a solvent such as water. Even in the case of diluting the solution, it is preferred that the pH of the solution after dilution achieve a pH of from 7.0 to 7.8, and that the concentrations of the anionic surfactant and the non-ionic surfactant in the solution after dilution achieve a final concentration of from 0.01 to 1 w/v%.

The calcium oxalate dihydrate crystals are preferably preserved at a preservation temperature of, for example, from 0 to 50°C. However, the preservation temperature is not particularly limited, as long as the octahedron shape of the calcium oxalate dihydrate crystals can be retained.

According to the preservation method according to the present embodiment, it is possible to stably preserve calcium oxalate dihydrate crystals over a long period of time. The calcium oxalate dihydrate crystals may be preserved for a preservation period of, for example, one or more weeks, one or more months, three or more months, six or more months, or one or more years.

### < Method for Detecting Calcium Oxalate Dihydrate Crystals in Urine Sample >

Another embodiment of the present disclosure is a method for detecting the presence of calcium oxalate dihydrate crystals in a urine sample derived from a subject, the method including the steps of:
analyzing the quality control liquid described in the section of < Quality Control Liquid > described above, using a urine sediment analyzer, in order to determine the form of calcium oxalate dihydrate crystals in the quality control liquid;
analyzing the urine sample derived from the subject, using the urine sediment analyzer;
comparing the form of the calcium oxalate dihydrate crystals in the quality control liquid, with the form of the calcium oxalate dihydrate crystals in the urine sample; and
determining that calcium oxalate dihydrate crystals are present in the urine sample, if the form of the calcium oxalate dihydrate crystals in the urine sample matches the form of the calcium oxalate dihydrate crystals in the quality control liquid.

The "subject" refers to an individual who undergoes the examination of urine sediments. The subject may be an individual with a disease, an individual suspected to have a disease, or a healthy individual. The subject is preferably a human being.

Whether or not the form of the calcium oxalate dihydrate crystals in the urine sample matches the form of the calcium oxalate dihydrate crystals in the quality control liquid can be automatically determined by the urine sediment analyzer to be used. For example, in the case of using the urine sediment analyzer disclosed in Patent Document 2, which is based on an unstained image analysis performed in a fluid, the analyzer that determines the type of a sediment in a urine sample, extracts feature values (such as shape, form, size and the like) from the images of the sediment; also extracts feature values from the images of the calcium oxalate dihydrate crystals in the quality control liquid; determines whether or not the forms of both match with each other, by the pattern matching of the respective feature values; and indicates that calcium oxalate dihydrate crystals are present in the urine sample when it is determined that the forms of both match with each other.

Further, the urine sediment analyzer may count the number of the calcium oxalate dihydrate crystals detected.

The definition of the urine sediment analyzer includes a urine sediment analyzer based on a microscope. The urine sediment analyzer based on a microscope may be, for example, AUTION EYE^{®} AI-4510 (manufactured by Arkray, Inc.).

By detecting the presence of calcium oxalate dihydrate crystals in a urine sample derived from a subject, or by the result of counting the number of the calcium oxalate dihydrate crystals in the urine sample derived from the subject, it is possible to suspect the presence of a disease such as urolithiasis in the subject, and to determine a treatment plan (for example, the administration of a drug) for such a disease, as necessary.

### < Method for Controlling Measurement Accuracy >

Another embodiment of the present disclosure is a method for controlling the measurement accuracy of an analyzer that measures the particle number concentration of calcium oxalate dihydrate crystals in a sample by counting the number of the calcium oxalate dihydrate crystals in the sample, the method including the steps of:
measuring the particle number concentration of calcium oxalate dihydrate crystals in the quality control liquid described in the section of the < Quality Control Liquid >, using the analyzer;
comparing the measured value of the particle number concentration with a reference value; and
determining the measurement accuracy based on the comparison between the measured value with the reference value.
The "particle number concentration" refers to the number of particles contained in a unit volume, which is also referred to as "number concentration".

The analyzer is, for example, an analyzer including a microscope, and a camera for imaging the particles in the sample. The analyzer acquires an image in which the particles in the sample are captured, by imaging the particles in the sample with the camera via the microscope; counts the number of particles (similar particles) which are captured in the acquired image and whose degree of similarity with the form of the calcium oxalate dihydrate crystals stored in the analyzer in advance is equal to or higher than a predetermined value, as calcium oxalate dihydrate crystals; and measures the particle number concentration of the similar particles in the sample as the particle number concentration of the calcium oxalate dihydrate crystals, based on the counted value. The analyzer that measures the particle number concentration of calcium oxalate dihydrate crystals in a sample may be, for example, a urine sediment analyzer based on a microscope, and the urine sediment analyzer based on a microscope may be, for example, AUTION EYE^{®} AI-4510 (manufactured by Arkray, Inc.).

The sample is not particularly limited as long as the sample possibly contains calcium oxalate dihydrate crystals. Examples of the sample include urine and blood. Of these, urine is preferred.

When the quality control liquid is analyzed using the analyzer, the calcium oxalate dihydrate crystals contained in the quality control liquid are counted as calcium oxalate dihydrate crystals, and the measured value of the particle number concentration of the calcium oxalate dihydrate crystals contained in the quality control liquid is obtained. The measurement accuracy of the analyzer may be determined by comparing the measured value with a reference value (also referred to as predetermined value), and based, for example, on the difference between the measured value and the reference value. In this case, the analyzer can be determined to have a good measurement accuracy, if the difference is less than a predetermined value (i.e., predetermined particle number concentration). In contrast, the analyzer can be determined to have a poor measurement accuracy, if the difference is equal to or higher than the predetermined value.

Further, the measurement accuracy of the analyzer may also be determined based on the proportion of the measured value of the particle number concentration of the calcium oxalate dihydrate crystals with respect to the reference value. In this case, the analyzer can be determined to have a good measurement accuracy, if the proportion is less than a predetermined value. In contrast, the analyzer can be determined to have a poor measurement accuracy, if the proportion is equal to or higher than the predetermined value. The predetermined value can be set as appropriate depending on the accuracy required for the analyzer.

The reference value (also referred to as "reference particle number concentration") can be, for example: the value obtained by analyzing the same quality control liquid with a plurality of analyzers to obtain a plurality of measured values of the particle number concentration of calcium oxalate dihydrate crystals, and averaging the thus obtained measured values; the measured value of the particle number concentration of calcium oxalate dihydrate crystals obtained by measuring the quality control liquid, by microscopy in which a laboratory technician observes a sample with a microscope and counts the number of sediments to measure the concentration of the sediments; the measured value of the particle number concentration of calcium oxalate dihydrate crystals obtained by measuring the quality control liquid with a quality-controlled analyzer; or the like. The reference value can also be the mean value of the particle number concentrations of calcium oxalate dihydrate crystals, which have been measured by analyzing the quality control liquid multiple times, in advance, with the above-described analyzer.

The accuracy of the microscopy can be controlled by obtaining the measured value of the particle number concentration of the calcium oxalate dihydrate crystals in the quality control liquid, by microscopy, and comparing the resulting measured value with the reference value.

### EXAMPLES

Examples are described for the purpose of disclosure, and are not intended to limit the scope of the present disclosure.

### Example 1 (Artificial Synthesis of Crystals)

At room temperature, an aqueous solution (6.3 mL) of 1,000 mg/dL human serum albumin (manufactured by Sigma-Aldrich Co. LLC.), a 100 mM aqueous solution (2.8 mL) of calcium chloride and a 100 mM aqueous solution (5.6 mL) of trisodium citrate dihydrate were mixed with distilled water (33.6 mL) in a beaker, to prepare a mixed solution 1. Ultrasound was applied to the beaker containing the mixed solution 1 at a frequency of 38 kHz, using an ultrasonic oscillator (ultrasonic washer, SONO CLEANER 50Z CA-2488Z (manufactured by KAIJO corporation)). A 100 mM aqueous solution of acetic acid was mixed with a 100 mM aqueous solution of sodium acetate at a volume ratio of 1:1, to prepare an acetate buffer solution. A 10 mM aqueous solution of potassium oxalate was mixed with a 2 M aqueous solution of NaCl in a beaker, and then the acetate buffer solution was added thereto to adjust the pH to 6.1, to obtain 14 mL of a mixed solution 2. While applying ultrasound at a frequency of 38 kHz with the above-described ultrasonic oscillator, the mixed solution 2 was added to the beaker containing the mixed solution 1, to prepare a mixed solution 3. Twenty minutes after the addition of the mixed solution 2, the ultrasonic oscillator was stopped, and the mixed solution 3 was left to stand for 10 minutes. The mixed solution 3 was transferred to a centrifuge tube, subjected to centrifugation (500 xg, 5 minutes), and the resulting supernatant was removed. Thereafter, 5 mL of an aqueous solution of LATEMUL^{®} PS (manufactured by Kao Corporation) at a final concentration of 0.5 w/v%, as an anionic surfactant, was added to the centrifuge tube. The above-described operation was repeated two more times, to obtain a precipitate containing calcium oxalate dihydrate crystals.

### Reference Example 1 (Collection of Natural Crystals, and Comparison with Artificial Crystals)

Thirty mL of urine (obtained from a hospital) collected from a patient was introduced into a centrifuge tube, subjected to centrifugation (500 xg, five minutes), and the resulting supernatant was removed. Thereafter, 5 mL of an aqueous solution of LATEMUL^{®} PS at a final concentration of 0.5 w/v%, as an anionic surfactant, was added to the centrifuge tube. The above-described operation was repeated two more times, to obtain a precipitate containing calcium oxalate dihydrate crystals.

The thus obtained precipitate containing calcium oxalate dihydrate crystals was mixed with 5 mL of PBS (pH 7.4) which is a liquid having a buffering capacity and which contains LATEMUL^{®} PS at a final concentration of 0.5 w/v%, as an anionic surfactant, to obtain a sample.

### Analysis with Urine Sediment Analyzer - Example 1 and Reference Example 1

The samples each containing the calcium oxalate dihydrate crystals prepared by the procedure described in Example 1 or Reference Example 1, the anionic surfactant and the liquid having a buffering capacity were analyzed by a urine sediment analyzer, AUTION EYE^{®} AI-4510 (manufactured by Arkray, Inc.). The images of the calcium oxalate dihydrate crystals of Example 1 obtained by the above-described operation are shown in FIG. 1, and the images of the calcium oxalate dihydrate crystals in urine of Reference Example 1 obtained by the above-described operation are shown in FIG. 2. It can be seen that the forms of both crystals of Example 1 and Reference Example 1 are extremely similar to each other, even by visual comparison.

### Example 2-1 (Preservation Stability with LATEMUL^{®} PS)

PBS having the composition shown in Table 1 below was prepared. A 40 w/v% aqueous solution of LATEMUL^{®} PS was mixed with the PBS such that the final concentration of LATEMUL^{®} PS was 0.1 w/v%, 0.5 w/v% or 1 w/v%, to prepare each aqueous solution (pH 7.4) of LATEMUL^{®} PS, to be used as each preservation liquid for calcium oxalate dihydrate crystals. The specific compositions of the respective preservation liquids are as shown in Table 2. The precipitate (prepared by the procedure described in Example 1) containing calcium oxalate dihydrate crystals was suspended in 5 mL of each of the thus prepared preservation liquids, to obtain each sample (liquid for control; hereinafter, referred to as "control liquid"). The pH of each sample is 7.4, which is the pH of the PBS. Each resulting sample (control liquid) contains the calcium oxalate dihydrate crystals (prepared by the procedure described in Example 1), LATEMUL^{®} PS (at a final concentration of 0.1 w/v%, 0.5 w/v% or 1 w/v%), which is an anionic surfactant, and the PBS (pH 7.4), which is a liquid having a buffering capacity. For each sample (control liquid), the state of the crystals was observed using a biological microscope BX-2700TL (manufactured by WRAYMER Inc.) in HPF mode (400 times), at time points immediately after the preparation of each sample, and one day, three days, seven days, two months, five months, six months and 20 months after allowing each sample to stand and to be preserved at room temperature. The results are shown in FIG. 3 and FIG. 4.

**Table 1. Composition of PBS (pH 7.4)**

| Component | Concentration (w/v%) |
|---|---|
| Sodium chloride | 0.800 |
| Disodium hydrogen phosphate | 0.144 |
| Potassium chloride | 0.020 |
| Potassium dihydrogen phosphate | 0.024 |

**Table 2. Composition of preservation liquid (PBS + LATEMUL^{®} PS) (pH 7.4)**

| Component | Preservation liquid (pH 7.4) | | |
|---|---|---|---|
| | PBS + 0.1 w/v% LATEMUL^{®} PS | PBS + 0.5 w/v% LATEMUL^{®} PS | PBS + 1.0 w/v% LATEMUL^{®} PS |
| LATEMUL^{®} PS | 0.100 w/v% | 0.500 w/v% | 1.000 w/v% |
| Sodium chloride | 0.798 w/v% | 0.790 w/v% | 0.780 w/v% |
| Disodium hydrogen phosphate | 0.144 w/v% | 0.142 w/v% | 0.140 w/v% |
| Potassium chloride | 0.020 w/v% | 0.020 w/v% | 0.020 w/v% |
| Potassium dihydrogen phosphate | 0.024 w/v% | 0.024 w/v% | 0.023 w/v% |

### Example 2-2 (No Surfactant Added)

The precipitate (prepared by the procedure described in Example 1) containing calcium oxalate dihydrate crystals was sufficiently washed with PBS to remove the surfactant, and then 5 mL of PBS (pH 7.4) alone as a liquid having a buffering capacity was added to the washed precipitate, to prepare a sample to which no surfactant was added. The state of the crystals was observed using a biological microscope BX-2700TL (manufactured by WRAYMER Inc.) in HPF mode (400 times), at time points immediately after the preparation of the sample, and one day and three days after allowing the sample to stand and to be preserved at room temperature. The results are shown in FIG. 3.

### Examination of Preservation Stability - Results

As can be seen from FIG. 3, calcium oxalate dihydrate crystals in all samples had an octahedron shape at the time of preparation. In the case of adding no surfactant, the aggregation of the calcium oxalate dihydrate crystals and the collapse of the octahedron shape of the crystals were observed one day after allowing the sample to stand. In contrast, in the case of adding LATEMUL^{®} PS as an anionic surfactant, no aggregation was observed and the octahedron shape of the crystals was retained even after three days, at all concentrations of LATEMUL^{®} PS.

As can be seen from FIG. 4, no aggregation was observed and the octahedron shape of the crystals was retained even after 20 months, at all concentrations of LATEMUL^{®} PS.

### Example 3 (Preservation Stability with Polyoxyethylene (20) Sorbitan Monolaurate)

The same procedure as in Example 2-1 was carried out, except that each aqueous solution of LATEMUL^{®} PS used as the preservation liquid in Example 2-1 was changed to an aqueous solution (at a final concentration of 0.01 w/v% or 0.1 w/v%) of polyoxyethylene (20) sorbitan monolaurate, which is a non-ionic surfactant. Specifically, polyoxyethylene (20) sorbitan monolaurate (Tween^{®} 20; manufactured by FUJIFILM Wako Pure Chemical Corporation) was mixed with the PBS such that the final concentration of polyoxyethylene (20) sorbitan monolaurate was 0.01 w/v% or 0.1 w/v%, to prepare each aqueous solution (pH 7.4) of polyoxyethylene (20) sorbitan monolaurate, to be used as each preservation liquid for calcium oxalate dihydrate crystals. The specific compositions of the respective preservation liquids are shown in Table 3. The precipitate (prepared by the procedure described in Example 1) containing calcium oxalate dihydrate crystals was suspended in 5 mL of each of the thus prepared preservation liquids, in the same manner as in Example 2-1, to obtain each sample (control liquid). The pH of each sample is 7.4, which is the pH of the PBS. Each resulting sample (control liquid) contains the calcium oxalate dihydrate crystals, the non-ionic surfactant, and the PBS as a liquid having a buffering capacity. The state of the crystals was observed using a biological microscope BX-2700TL (manufactured by WRAYMER Inc.) in HPF mode (400 times), at time points immediately after the preparation of each sample (control liquid), and two days and five days after allowing each sample (control liquid) to stand and to be preserved at room temperature. The results are shown in FIG. 5 and FIG. 6.

**Table 3. Composition of preservation liquid (PBS + Polyoxyethylene (20) sorbitan monolaurate) (pH 7.4)**

| Component | Preservation liquid (pH 7.4) | |
|---|---|---|
| | PBS + 0.01 w/v% Polyoxyethylene (20) sorbitan monolaurate | PBS + 0.1 w/v% Polyoxyethylene (20) sorbitan monolaurate |
| Polyoxyethylene (20) sorbitan monolaurate | 0.010 w/v% | 0.100 w/v% |
| Sodium chloride | 0.800 w/v% | 0.799 w/v% |
| Disodium hydrogen phosphate | 0.144 w/v% | 0.144 w/v% |
| Potassium chloride | 0.020 w/v% | 0.020 w/v% |
| Potassium dihydrogen phosphate | 0.024 w/v% | 0.024 w/v% |

As can be seen from FIG. 5, the form of the calcium oxalate dihydrate crystals was retained even after two days, in the case of containing polyoxyethylene (20) sorbitan monolaurate as the non-ionic surfactant.

As can be seen from FIG. 6, the octahedron shape of the crystals was not retained after five days, at either concentration of polyoxyethylene (20) sorbitan monolaurate.

### Example 4 (Preservation Stability with Sodium Dodecyl Sulfate)

The same procedure as in Example 2-1 was carried out, except that each aqueous solution of LATEMUL^{®} PS used as the preservation liquid in Example 2-1 was changed to an aqueous solution (at a final concentration of 0.1 w/v%, 0.5 w/v% or 1 w/v%) of sodium dodecyl sulfate (SDS), which is an anionic surfactant. Specifically, sodium dodecyl sulfate (SDS; manufactured by FUJIFILM Wako Pure Chemical Corporation) was mixed with the PBS such that the final concentration of SDS was 0.1 w/v%, 0.5 w/v% or 1.0 w/v%, to prepare each aqueous solution (pH 7.4) of SDS, to be used as each preservation liquid for calcium oxalate dihydrate crystals. The specific compositions of the respective preservation liquids are shown in Table 4. The precipitate (prepared by the procedure described in Example 1) containing calcium oxalate dihydrate crystals was suspended in 5 mL of each of the thus prepared preservation liquids, in the same manner as in Example 2-1, to obtain each sample (control liquid). The pH of each sample is 7.4, which is the pH of the PBS. Each resulting sample (control liquid) contains the calcium oxalate dihydrate crystals, and the PBS as a liquid having a buffering capacity. The state of the crystals was observed using a biological microscope BX-2700TL (manufactured by WRAYMER Inc.) in HPF mode (400 times), at time points immediately after the preparation of each sample (control liquid), and two days, five days, eight days and nine months after allowing each sample (control liquid) to stand and to be preserved at room temperature. The results are shown in FIG. 7.

**Table 4. Composition of preservation liquid (PBS + SDS) (pH 7.4)**

| Component | Preservation liquid (pH 7.4) | | |
|---|---|---|---|
| | PBS + 0.1 w/v% SDS | PBS + 0.5 w/v% SDS | PBS + 1.0 w/v% SDS |
| SDS | 0.100 w/v% | 0.500 w/v% | 1.000 w/v% |
| Sodium chloride | 0.799 w/v% | 0.796 w/v% | 0.792 w/v% |
| Disodium hydrogen phosphate | 0.144 w/v% | 0.143 w/v% | 0.142 w/v% |
| Potassium chloride | 0.020 w/v% | 0.020 w/v% | 0.020 w/v% |
| Potassium dihydrogen phosphate | 0.024 w/v% | 0.024 w/v% | 0.024 w/v% |

As can be seen from FIG. 7, the octahedron shape of the calcium oxalate dihydrate crystals was retained at all final concentrations until after eight days. After nine months, however, the octahedron shape of the crystals was retained only in a sample having a final concentration of 0.5 w/v%.

The evaluation results of the preservation stability of the calcium oxalate dihydrate crystals in the PBS containing LATEMUL^{®} PS, polyoxyethylene (20) sorbitan monolaurate or SDS are shown in Table 5. Those in which calcium oxalate dihydrate crystals were dispersed without aggregation and able to retain their octahedron shape were evaluated as "∘", and those in which the crystals aggregated or failed to retain their octahedron shape were evaluated as "×". Further, the period of time during which the crystals were confirmed to be dispersed and to retain their octahedron shape, was defined as the number of days capable of being preserved.

**Table 5. Evaluation Results of preservation stability of calcium oxalate dihydrate crystals**

| | Immediately after preparation | 1 day later | 2 days later | 3 days later | 5 days later | 7 days later | 8 days later |
|---|---|---|---|---|---|---|---|
| PBS alone | ○ | ○ | N/A | × | × | × | N/A |
| PBS + 0.1 w/v% LATEMUL^{®} PS | ○ | ○ | N/A | ○ | N/A | ○ | N/A |
| PBS + 0.5 w/v% LATEMUL^{®} PS | ○ | ○ | N/A | ○ | N/A | ○ | N/A |
| PBS + 1.0 w/v% LATEMUL^{®} PS | ○ | ○ | N/A | ○ | N/A | ○ | N/A |
| PBS + 0.01 w/v% Polyoxyethylene (20) sorbitan monolaurate | ○ | N/A | ○ | N/A | × | N/A | N/A |
| PBS + 0.1 w/v% Polyoxyethylene (20) sorbitan monolaurate | ○ | N/A | ○ | N/A | × | N/A | N/A |
| PBS + 0.1 w/v% SDS | ○ | N/A | ○ | N/A | ○ | N/A | ○ |
| PBS + 0.5 w/v% SDS | ○ | N/A | ○ | N/A | ○ | N/A | ○ |
| PBS + 1.0 w/v% SDS | ○ | N/A | ○ | N/A | ○ | N/A | ○ |

| | | 2 months later | 5 months later | 6 months later | 9 months later | 20 months later | Number of days capable of being preserved |
|---|---|---|---|---|---|---|---|
| PBS alone | | × | × | × | N/A | × | 1 day |
| PBS + 0.1 w/v% LATEMUL^{®} PS | | ○ | ○ | ○ | N/A | ○ | 20 or more months |
| PBS + 0.5 w/v% LATEMUL^{®} PS | | ○ | ○ | ○ | N/A | ○ | 20 or more months |
| PBS + 1.0 w/v% LATEMUL^{®} PS | | ○ | ○ | ○ | N/A | ○ | 20 or more months |
| PBS + 0.01 w/v% Polyoxyethylene (20) sorbitan monolaurate | | N/A | N/A | N/A | N/A | N/A | 2 days |
| PBS + 0.1 w/v% Polyoxyethylene (20) sorbitan monolaurate | | N/A | N/A | N/A | N/A | N/A | 2 days |
| PBS + 0.1 w/v% SDS | | N/A | N/A | N/A | × | N/A | 8 days |
| PBS + 0.5 w/v% SDS | | N/A | N/A | N/A | ○ | N/A | 9 or more months |
| PBS + 1.0 w/v% SDS | | N/A | N/A | N/A | × | N/A | 8 days |

| | | | | | | | |
|---|---|---|---|---|---|---|---|
| ∘: Calcium oxalate dihydrate crystals were dispersed and able to retain their octahedron shape. ×: Calcium oxalate dihydrate crystals aggregated or failed to retain their octahedron shape. N/A: Not measured | | | | | | | |

The number of days capable of being preserved of the calcium oxalate dihydrate crystals in the PBS to which no surfactant was added was one day, whereas the number of days capable of being preserved of the crystals in each PBS to which a surfactant was added was two or more days. Further, the number of days capable of being preserved of the crystals in each PBS containing LATEMUL^{®} PS at a final concentration of 0.1 w/v%, 0.5 w/v% or 1.0 w/v%, and the number of days capable of being preserved of the crystals in each PBS containing SDS at a final concentration of 0.1 w/v%, 0.5 w/v% or 1.0 w/v%, were both eight or more days. The number of days capable of being preserved of the crystals in each PBS containing LATEMUL^{®} PS at a final concentration of 0.1 w/v%, 0.5 w/v% or 1.0 w/v%, and the number of days capable of being preserved of the crystals in the PBS containing SDS at a final concentration of 0.5%, were both nine or more months. Further, the number of days capable of being preserved of the crystals in each PBS containing LATEMUL^{®} PS at a final concentration of 0.1 w/v%, 0.5 w/v% or 1.0 w/v%, was 20 or more months.

These results have revealed that the quality control liquid containing calcium oxalate dihydrate crystals preferably contains an anionic surfactant or a non-ionic surfactant. In other words, it has been found out that the quality control liquid is preferably a suspension in which calcium oxalate dihydrate crystals are suspended in a solution of an anionic surfactant or a non-ionic surfactant. The results have also revealed that the quality control liquid more preferably contains an anionic surfactant at a final concentration of 0.1 w/v%, 0.5 w/v% or 1.0 w/v%, and specifically, preferably contains LATEMUL^{®} PS or SDS at such a final concentration. Further, it has been found out that the quality control liquid still more preferably contains LATEMUL^{®} PS at a final concentration of 0.1 w/v%, 0.5 w/v% or 1.0 w/v%, or contains SDS at a final concentration of 0.5 w/v%. Moreover, it has been found out that the quality control liquid still more preferably contains LATEMUL^{®} PS at a final concentration of 0.1 w/v%, 0.5 w/v% or 1.0 w/v%. The results have also revealed that the solution of an anionic surfactant or a non-ionic surfactant is useful as a solution to be used for preserving calcium oxalate dihydrate crystals.

It is noted that the preservation liquid, the preservation method and the quality control liquid according to the present disclosure can be applied not only to calcium oxalate dihydrate crystals which have been artificially produced as in Example 1, but also to calcium oxalate dihydrate crystals which have been collected from human urine as in Reference Example 1.

## Claims

1. Use of a solution comprising an anionic surfactant or a non-ionic surfactant for preserving calcium oxalate dihydrate crystals.

2. The use according to claim 1, wherein the solution has a pH of from 7.0 to 7.8.

3. The use according to claim 1 or claim 2, wherein the solution further comprises a phosphoric salt.

4. The use according to any one of claims 1 to 3, wherein the anionic surfactant or the non-ionic surfactant has a final concentration of from 0.01 to 1 w/v% in said solution.

5. The use according to any one of claims 1 to 4, wherein the anionic surfactant is an alkane sulfonate salt.

6. The use according to any one of claims 1 to 4, wherein the anionic surfactant is a dodecyl sulfate salt.

7. The use according to any one of claims 1 to 4, wherein the non-ionic surfactant is a polyoxyethylene sorbitan fatty acid ester.

8. A method for preserving calcium oxalate dihydrate crystals, the method comprising preserving the calcium oxalate dihydrate crystals in a solution comprising an anionic surfactant or a non-ionic surfactant.

9. The method according to claim 8, wherein the anionic surfactant is an alkane sulfonate salt.

10. The method according to claim 8, wherein the anionic surfactant is a dodecyl sulfate salt.

11. The method according to claim 8, wherein the non-ionic surfactant is a polyoxyethylene sorbitan fatty acid ester.

12. A method of controlling a measurement accuracy of an analyzer that measures a particle number concentration of calcium oxalate dihydrate crystals in a sample, the method including the steps of:
measuring a particle number concentration of calcium oxalate dihydrate crystals in a solution comprising a predetermined particle number concentration of the calcium oxalate dihydrate crystals using the analyzer;
comparing the measured value of the particle number concentration with the predetermined particle number concentration; and
determining the measurement accuracy based on the comparison between the measured value with the predetermined particle number concentration,
wherein the solution further comprises an anionic surfactant or a non-ionic surfactant.

13. The method according to claim 12, wherein the calcium oxalate dihydrate crystals include octahedron-shaped crystals.

## Patentansprüche

1. Verwendung einer Lösung, die ein anionisches Tensid oder ein nicht-anionisches Tensid umfasst, zum Konservieren für Calciumoxalatdihydratkristalle.

2. Verwendung gemäß Anspruch 1, wobei die Lösung einen pH von 7,0 bis 7,8 aufweist.

3. Verwendung gemäß Anspruch 1 oder Anspruch 2, wobei die Lösung ferner ein Phosphorsalz umfasst.

4. Verwendung gemäß einem der Ansprüche 1 bis 3, wobei das anionische Tensid oder das nicht-anionische Tensid eine Endkonzentration von 0,01 bis 1 w/v% in der genannten Lösung aufweist.

5. Verwendung gemäß einem der Ansprüche 1 bis 4, wobei das anionische Tensid ein Alkansulfonatsalz ist.

6. Verwendung gemäß einem der Ansprüche 1 bis 4, wobei das anionische Tensid ein Dodecylsulfatsalz ist.

7. Verwendung gemäß einem der Ansprüche 1 bis 4, wobei das nicht-anionische Tensid ein Polyoxyethylensorbitan-Fettsäureester ist.

8. Verfahren zum Konservieren von Calciumoxalatdihydratkristallen, wobei das Verfahren das Konservieren der Calciumoxalatdihydratkristalle in einer Lösung, die ein anionisches Tensid oder ein nicht-anionisches Tensid umfasst, umfasst.

9. Verfahren gemäß Anspruch 8, wobei das anionische Tensid ein Alkansulfonatsalz ist.

10. Verfahren gemäß Anspruch 8, wobei das anionische Tensid ein Dodecylsulfatsalz ist.

11. Verfahren gemäß Anspruch 8, wobei das nicht-anionische Tensid ein Polyoxyethylensorbitan-Fettsäureester ist.

12. Verfahren zum Steuern einer Messgenauigkeit eines Analysators, der eine Partikelanzahlkonzentration von Calciumoxalatdihydratkristallen in einer Probe misst, wobei das Verfahren die folgenden Schritte einschließt:
Messen einer Partikelanzahlkonzentration von Calciumoxalatdihydratkristallen in einer Lösung, die eine vorherbestimmte Partikelanzahlkonzentration der Calciumoxalatdihydratkristalle umfasst, unter Verwendung des Analysators;
Vergleichen des gemessenen Werts der Partikelanzahlkonzentration mit der vorherbestimmten Partikelanzahlkonzentration; und
Bestimmen der Messgenauigkeit basierend auf dem Vergleich zwischen dem gemessenen Wert und der vorherbestimmten Partikelanzahlkonzentration,
wobei die Lösung ferner ein anionisches Tensid oder ein nicht-anionisches Tensid umfasst.

13. Verfahren gemäß Anspruch 12, wobei die Calciumoxalatdihydratkristalle oktaederförmige Kristalle einschließen.

## Revendications

1. Utilisation d'une solution comprenant un tensioactif anionique ou un tensioactif non ionique pour la conservation de cristaux d'oxalate de calcium dihydraté.

2. Utilisation selon la revendication 1, dans laquelle la solution a un pH de 7,0 à 7,8.

3. Utilisation selon la revendication 1 ou la revendication 2, dans laquelle la solution comprend en outre un sel phosphorique.

4. Utilisation selon l'une quelconque des revendications 1 à 3, dans laquelle le tensioactif anionique ou le tensioactif non ionique a une concentration finale de 0,01 à 1 % p/v dans ladite solution.

5. Utilisation selon l'une quelconque des revendications 1 à 4, dans laquelle le tensioactif anionique est un sel sulfonate d'alcane.

6. Utilisation selon l'une quelconque des revendications 1 à 4, dans laquelle le tensioactif anionique est un sel dodécylsulfate.

7. Utilisation selon l'une quelconque des revendications 1 à 4, dans laquelle le tensioactif non ionique est un ester d'acide gras de polyoxyéthylène sorbitane.

8. Procédé de conservation de cristaux d'oxalate de calcium dihydraté, le procédé comprenant la conservation des cristaux d'oxalate de calcium dihydraté dans une solution comprenant un tensioactif anionique ou un tensioactif non ionique.

9. Procédé selon la revendication 8, dans lequel le tensioactif anionique est un sel sulfonate d'alcane.

10. Procédé selon la revendication 8, dans lequel le tensioactif anionique est un sel dodécylsulfate.

11. Procédé selon la revendication 8, dans lequel le tensioactif non ionique est un ester d'acide gras de polyoxyéthylène sorbitane.

12. Procédé de contrôle de la précision de mesure d'un analyseur qui mesure une concentration en nombre de particules de cristaux d'oxalate de calcium dihydraté dans un échantillon, le procédé comprenant les étapes qui consistent à :
mesurer une concentration en nombre de particules de cristaux d'oxalate de calcium dihydraté dans une solution comprenant une concentration en nombre de particules prédéterminée des cristaux d'oxalate de calcium dihydraté en utilisant l'analyseur ;
comparer la valeur mesurée de la concentration en nombre de particules avec la concentration en nombre de particules prédéterminée ; et
déterminer la précision de mesure sur la base de la comparaison entre la valeur mesurée et la concentration en nombre de particules prédéterminée,
dans lequel la solution comprend en outre un tensioactif anionique ou un tensioactif non ionique.

13. Procédé selon la revendication 12, dans lequel les cristaux d'oxalate de calcium dihydraté comprennent des cristaux octaédriques.
